# EUROPEAN PATENT SPECIFICATION

(11) **EP 1 250 329 B1**
(45) Date of publication and mention of the grant of the patent: **03.02.2010**
(21) Application number: 00977769.9
(22) Date of filing: 01.12.2000
(51) Int. Cl.: C07D 279/20, B01J 2/04

(54) **PHENOTHIAZINES IN PRILL FORM**
PHENOTHIAZINE ALS PRILL-GRANULATE
PHENOTHIAZINES EN FORME DE GRANULE

(30) Priority: 03.12.1999 US 453685
(43) Date of publication of application: 23.10.2002
(73) Proprietor: Cytec Industries Inc., West Paterson, NJ 07424 (US)
(72) Inventor: VANZIN, David, Alan, Mount Pleasant, TN 38474 (US)
(74) Representative: Destryker, Elise Martine
(86) International application number: PCT/GB2000/004600
(87) International publication number: WO 2001/040209

(56) References cited:
- EP-A- 0 070 436
- EP-A- 0 275 910
- EP-A- 0 277 508
- EP-A- 0 499 126
- US-A- 3 951 638

## Description

### Background to the Invention

Phenothiazine is an aromatic amine-based product used in a wide variety of applications, including as an inhibitor, antioxidant and short-stopping agent in a variety of diverse applications such as the stabilization of acrylic acids, esters and monomers or as a stabilizer for chloroprene monomer, styrene monomer and other vinylic monomers; as an antioxidant in synthetic lubricants and oils, in polyols for polyurethanes and polyester and vinyl ester resins; and as a pharmaceutical intermediate.

Phenothiazine is typically manufactured in flake and powder forms and having a bright yellow appearance (indicating lack of oxidation). The flake form may be prepared by coating molten phenothiazine onto a drum flaker, whereupon is is cooled and crystallised into a thin layer which is scraped off as a mixture of flakes and fines (powder). The product is then conveyed to a physical separation (classification) process in which the fines (powder) are separated from the flakes, typically by the use of sizing screens. The flaked phenothiazine is then packaged and shipped to customers who convey or transfer this through their own processing equipment. Despite classification, the product generally contains up to about 6% of fines after manufacture. Furthermore, the flakes are prone to breakdown into fines during subsequent shipping and handling.

The generation and presence of fines (powder) in flaked phenothiazine presents problems. Flaked products containing such fines suffer from the deficiencies of non-uniform particle size, caking, dustiness and clumping. Phenothiazine, being a respiratory, skin, eye and gastrointestinal irritant and skin sensitizer, is more likely to cause problems in a finely-divided form. Fines in the flaked product also increase the likelihood of explosion. Flaked phenothiazine containing high levels of fines (i.e., more than about 6% particles having diameters less than 500 microns) is especially prone to caking or clumping.

Non-uniform particle size in flaked phenothiazine increases the tendency of the product to cake and/or clump and to resist ready flow transfer both internally and at customer facilities. The caking and/or clumping makes it difficult to discharge the phenothiazine from containers such as bins, bags, trucks, storage silos and the like and difficult to transfer. It can also cause the formation of bridges or blocks in containers. Fines also present safety, health and environmental issues. From a safety standpoint, fines are of particular concern due to the increased risk of explosion, as well as increased risk to employee health, especially through skin irritation and skin sensitization and the like, as noted above.

Conventional prilling technology requires molten material to be passed through an orifice opening after which it cools and solidifies into a prill as it falls through the air.

Typically, prilling towers are 80 to 300 feet in height and require large volumes of air. Drawbacks with conventional prilling technology include the fact that large towers and significant volumes of air are required. This requires high capital costs and significant operational costs. Additionally, phenothiazine currently cannot be prilled by conventional technology as the product in molten state, reacts and undergoes oxidation, changing in both chemical composition and color. An oxidized form of phenothiazine is green to grey in color. This form is of differing chemical composition to the desired pure phenothiazine. Purified phenothiazine in a non-oxidized form is a bright yellow color.

The melting temperature of phenothiazine is 184 °C and, thus, its handling and transfer in the molten state is difficult. Pumping molten phenothiazine the 80 to 300 feet, as would be required to reach the top of a conventional prilling tower, is difficult without significant heat tracing of lines. If line temperatures drop below the melting point of the product, it quickly solidifies in the transfer line, causing operational difficulties.

In accordance with the foregoing, there is a need in the art for a method of production which reduces the problems associated with significant levels of fines in the finished phenothiazine, but which still produces high quality material. There is also a need in the art to reduce the problems associated with caking and/or clumping of phenothiazine during shipment, transfer and storage.

With regard to quality, it is highly desirable that any new method produces a product which meets the industry-wide quality specification of ~ 99.6 % phenothiazine in the product. However, color is also an important indicator of purity and it is highly desirable that the product color is from a pale-greenish-yellow through to bright yellow and especially is free from any grey or completely green material, which can adversely affect downstream applications, even if analytical purity is ~ 99.6 %. Phenothiazine color is conveniently assessed by use of a Munsell Color Chart and it is highly desirable that the appearance of the product falls within the region of the Munsell Color Chart defined by Hue Symbol 5Y and Color Spaces: 8/4 to 8/12, 8.5/4 to 8.5/12 and 9/4 to 9/8, inclusive, and more especially in the region defined by Hue Symbol 5Y and Color Spaces 8.5/8 to 8.5/12 and 9/6 to 9/8.

### Brief Summary of the Invention

The invention includes solid phenothiazine or an analog or derivative thereof of formula II infra (as hereinafter described), comprising a plurality of prills, wherein said prills are generally spherical and have an average diameter of from 0.5 mm to 2.3 mm, and wherein the solid products contain a mass fraction of not more than 6 % of fines, i. e., of particles having diameters of less than 500 µm.

In the following description and claims, phenothiazine refers to the compound of Formula (I) (see below) and phenothiazine material refers generically to phenothiazine of Formula (I) and/or an analog or derivative of phenothiazine of Formula (II) (see below).

The invention further includes a method of reducing the level of fines (powder) in phenothiazine material, comprising forming the phenothiazine material in prill form such that the prills have a generally spherical shape.

The invention also includes solid phenothiazine material comprising a plurality of prills of phenothiazine material, wherein the prills are generally spherical and the product has no greater than about 6% by weight fines (i.e. particles with diameters <500µ).

The invention includes a method for making prills of phenothiazine material comprising introducing molten phenothiazine material into at least one nozzle having a plurality of holes to form molten droplets of phenothiazine material and cooling the droplets to form solid prills, all within an inert environment. The method is especially suitable for making phenothiazine in the form of greenish-yellow to yellow prills. The invention also includes solid phenothiazine material formed by that method.

The invention is directed to phenothiazine material having improved properties and reduced levels of fines (powder) which is in prill form as well as to a method for making the phenothiazine material in prill form. The invention is further directed to a method for reducing the level of fines in phenothiazine material while maintaining product quality. In all aspects of the invention it is preferred that the phenothiazine material is phenothiazine.

According to a first aspect of the invention there is provided a solid phenothiazine material in the form of a plurality of generally spherical prills.

In the case of phenothiazine, the prill form preferably has a greenish-yellow to yellow color, more preferably a color within the region of the Munsell Color Chart defined by Hue Symbol 5Y and Color Spaces: 8/4 to 8/12, 8.5/4 to 8.5/12 and 9/4 to 9/8, inclusive, more especially in the region defined by Hue Symbol 5Y and Color Spaces: 8.5/8 to 8.5/12 and 9/6 to 9/8, inclusive. It is especially preferred that the phenothiazine prill-form has a bright yellow color, more especially a bright yellow color equivalent to that of flaked phenothiazine.

The phenothiazine prill product preferably contains at least 99.6% phenothiazine.

According to another aspect of the present invention there is provided a method for making solid phenothiazine material in prill form comprising breaking a stream of molten phenothiazine material into a plurality of evenly sized droplets and cooling the droplets to form solid phenothiazine material in the form of prills, the phenothiazine material being maintained within an inert gas (preferably nitrogen) environment while in the molten state. It is further preferred that the inert gas environment is maintained until the phenothiazine material, in prill form, has cooled to below about 140°C.

### Detailed Description of the Invention

The prills may be formed by feeding molten phenothiazine material into at least one nozzle having a plurality of holes to form molten droplets of phenothiazine material which are cooled to form prills. The molten phenothiazine material may be any available form of phenothiazine material, such as powder or flake.

Phenothiazine is a solid material which is currently commercially available in both flake and powder forms. As used herein, phenothiazine material may include any phenothiazine analog or derivative, provided it is in solid form and can be made to be in a molten form, for example through application of heat.

Phenothiazine has a molecular weight of 199.26 and a chemical formula of C₁₂H₉NS. A typical commercially-available phenothiazine has a melting point of 184°C and a boiling point of 371°C. The bulk density is about 0.85 for flake product and about 0.75 for powder. The chemical formula of phenothiazine is as follows:

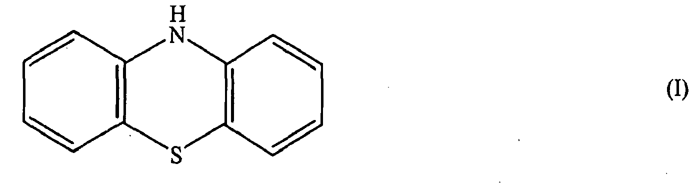

Phenothiazine material includes phenothiazine and its analogs and derivatives including, without limitation, compounds having formula (II): 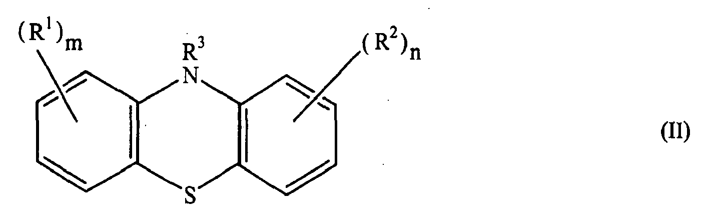 wherein R¹, R², and R³ may be the same or different and may be hydrogen; halogens, such as chlorine and fluorine and the like; branched or straight chain, and substituted or unsubstituted hydrocarbon groups such as alkyl, alkenyl, or alkynyl groups of from 1 to 26 carbon atoms; substituted and unsubstituted aryl groups and aralkyl groups; or functional groups, including, but not limited to sulfonyl, carboxy, amine, alkylamine, hydroxy, carboxy, silyl, siloxy; and other similar derivatives and their salts. Substituents for the aforementioned hydrocarbon, aryl and aralkyl groups may include any of the above functional groups as well as inter-chain elements such as oxygen, sulfur, silicon, nitrogen, and the like. Most preferably each of R¹, R² and R³ in Formula (II) is hydrogen. In formula (II), m and n are preferably independently from about 1 to about 4.

To form the prills, the phenothiazine material in molten form, which may be phenothiazine in accordance with formula (I) and/or phenothiazine derivatives or analogs in accordance with formula (II), is fed into the nozzle chamber of a jet priller capable of receiving a molten phenothiazine material and passed through prilling nozzle(s) having a plurality of holes.

Understanding of the foregoing summary and the detailed description of the invention, including preferred embodiments thereof, may be assisted by their consideration in conjunction with the appended drawing. For the purpose of illustrating the invention, there is shown in the drawing a preferred embodiment. It should be understood, however, that the invention is not limited to the precise arrangements and instrumentalities shown in the drawing. In the drawing,

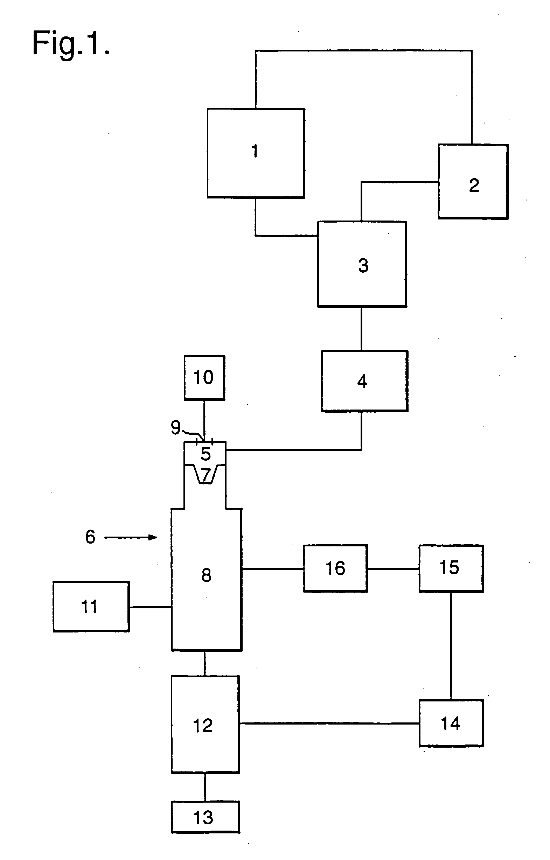

Figure 1 is a schematic representation of a jet priller and associated equipment suitable for making phenothiazine prills in accordance with the invention. A method for forming phenothiazine prills in accordance with the invention is described with reference to Fig. 1.

Phenothiazine is conveniently converted into the molten state by heating, to a temperature from about 190°C to about 215°C, more preferably from about 205°C to about 215°C, in a Feed Tank (1) under an inert atmosphere (preferably nitrogen gas) provided from a pressurised Inert Gas Store (2). The molten mass is then transferred by pressure of inert gas to a Conditioning Vessel (3) where the temperature is adjusted to about 195-200°C. The inert gas (preferably nitrogen) pressure is maintained at about 1.5 to 3 bar in the Conditioning Vessel (3) in order to drive the molten phenothiazine into the Nozzle Chamber (5) of the Jet Priller (6) under a nitrogen atmosphere, via a Filter (4) to remove foreign material. The inert gas pressure in the Nozzle Chamber (5) is carefully controlled at a fixed pressure preferably between 0 and 1 bar, and more preferably from 0.035 to 0.80 bar, in order to control the flow rate of phenothiazine through the Prilling Nozzle (7). It has been observed that feeding the molten phenothiazine to the Nozzle Chamber at a temperature higher than about 215°C and/or operating at a pressure above about 1 bar in the Nozzle Chamber (5) causes the molten phenothiazine to break up in a less controlled manner, former more fine droplets, thus and resulting in more fines in the final product.

As the molten phenothiazine passes through the holes of the Prilling Nozzle (7), into the top of the Prilling Column (8), it is broken in a controlled manner into droplets of from about 1 mm to about 2 mm, by the action of a Vibrating Membrane (9) in the wall of the Nozzle Chamber (5) which is vibrated by Vibration Unit (10) operating at a frequency from about 100. to about 1500 Hz, more preferably from about 400 to about 1100 Hz. A stream of liquefied inert gas, especially liquid nitrogen, is simultaneously fed into the side of the Prilling Column (8) from a Liquefied Inert Gas Store (11) and the mixture of cold inert gas, in liquid and gaseous forms, quickly cools the small droplets of molten phenothiazine to generally spherical, prills. The use of cold inert gas, especially nitrogen, is important in order to maintain product quality and to achieve a greenish yellow to yellow colored product. Although other inert cooling gases, may be used to form phenothiazine prills according to the invention, nitrogen is preferred, because it is so effective in rapidly cooling the prills and preventing any contact with an oxidising atmosphere, thus maintaining the preferred yellow color, especially a color in the range Hue Symbol: 5Y Color Space: 8/4 to 8/12, 8.5/4 to 8.5/12 and 9/4 to 9/8, inclusive.

The droplets are cooled initially by the mixture of cold, gaseous and liquid inert gas (especially nitrogen) into partially crystalline prills as they fall through the Prilling Column (8). By the time the prills reach the foot of the Prilling Column (8), they are generally at a temperature from about 120°C to about 170°C, more preferably about 140°C, from where they pass into a Spiral Cooler (12), having the form of a cyclone, wherein, the prills cool further in the cold inert gas environment, until crystallisation is complete, by the time they reach the bottom of the Spiral Cooler (12). Here they are removed from the Jet Priller (6), via an Air-lock (13) to prevent ingress of air, while the inert gas carrying any fines, which may be inadvertently formed during passage through the nozzles, passes out of the Spiral Cooler (12) through a side duct.

It has been found that the quality of the phenothiazine prills is sensitive to the relative flow rates of phenothiazine and inert gas. The flow rate of nitrogen through the Prilling Column (8) and Spiral Cooler (12) is preferably from 0.25 to 0.3 kg per kg of prilled phenothiazine.

The inert gas, leaving the Spiral Cooler is drawn by a Fan (15) into a Cyclone (14) to remove fines and recycled into the Prilling Column (8) via a Cooler (16) to supplement the feed of fresh liquid nitrogen.

A preferred jet priller is commercially available from GMF Gouda, Goudsche Machinefabriek, B.V. in Waddinzveen, Holland, sold as Model JP15 and prills can be manufactured using such a jet priller.

Phenothiazine in prill form is useful for many applications, particularly those in which phenothiazine powder is problematic. The phenothiazine prills in accordance with the invention may be used in a wide variety of applications, including as a stabilizer for a variety of chemical applications. The product may also be used for an inhibitor, antioxidant and short-stopping agent in a variety of diverse applications such as the stabilization of acrylic acids, esters and monomers or as a stabilizer for chloroprene monomer, styrene monomer and other vinylic monomers. The prill product is also useful as an antioxidant in synthetic lubricants and oils, polyols for polyurethanes and polyester and vinyl ester resins. Due to its high level of activity, phenothiazine functions at very low concentrations and enhances the functioning of other stabilizers. It also functions well in strongly acidic environments as well as in both air or nitrogen environments. Phenothiazine material, in prill form, is also a useful pharmaceutical intermediate.

The prilled phenothiazine material of the invention preferably has less than about 6% and more preferably less than about 1% by weight fines (powder), i.e. particles having diameters less than about 500 microns. Further, the trilled phenothiazine material preferably has an average diameter as measured in the longest dimension of the prill product from about 0.5 mm to about 2.3 mm and more preferably from about 1 mm to about 2 mm. The generally spherical prills of phenothiazine material in accordance with the invention have significantly improved flow characteristics due to their uniformity of size and are generally safer in use than standard phenothiazine material in flake or powder form.

The invention will now be described in further detail with respect to the following non-limiting examples.

### Example 1

### Small-scale trial in a simulated laboratory-scale Driller

A one liter, 3-neck reactor flask was used to simulate a flaker feed tank. It was equipped with nitrogen feed, a stirrer for agitation, a temperature probe and a bottom outlet. The apparatus was vacuum evacuated and nitrogen purged three times prior to product loading under nitrogen. The reactor was then charged with phenothiazine flake which was heated under nitrogen to a molten state at about 200°C. The molten phenothiazine was then slowly dripped through the bottom outlet valve (a simulated nozzle) into approximately one liter of liquid nitrogen (at about -192°C) in a vacuum jacketed Dewar flask. Bright yellow prills were formed and subjected to selective analyses. The bright yellow prills met all product specifications, evidenced no precipitate, and handled in a similar manner and efficiency to standard phenothiazine flake.

### Example 2

### Large scale trial in a commercial jet priller (see Figure 1)

Phenothiazine flake was charged to a Feed Tank (1). The flake was then melted and conveyed at about 200°C, under 3.1 bars pressure, (nitrogen gas) to a Conditioning Tank (3). The product was then fed under nitrogen pressure into the Nozzle Chamber (5) of a Model JP15/1 Closed-Loop jet priller from GMF Gouda (6) having a Nozzle (7) with 100 holes each having a diameter of about 0.5 mm. The phenothiazine flow rate through the Nozzle (7) was controlled by maintaining a constant nitrogen pressure in the Nozzle Chamber (5). The product was broken into 1-2 mm droplets as it passed through the nozzle (7), by means of a Vibrating Membrane (9) in the wall of the Nozzle Chamber (5), controlled, with the aid of a stroboscope, at a frequency of 1005-1007 Hz. Prills were formed from the droplets by immediate liquid nitrogen cooling of the molten droplet into a solid prill form. The molten feed was at approximately 200°C (ranging generally from about 194.7 to 195.5°C) and the liquid nitrogen temperature was about -192°C. The flow rate of liquid nitrogen from Liquefied Inert gas Store (11) was varied from 0.25 kg of nitrogen per kg of prills to 0.30 kg of nitrogen per kg of prills. The product solidified into a prill as it fell through the liquid/nitrogen gas environment in the cryogenic Prilling Column (8). Further cooling occurred in the Spiral Cooler (12), after which the product was discharged from the Jet Priller (6) via Air-lock (13). The nitrogen gas, containing any generated fines, was drawn by Fan (15) through Cyclone (14) to remove the fines and the nitrogen gas re-circulated back into the Prilling Column (8) after passing through Cooler (16). The trial resulted in the production of greenish-yellow (Munsell Color Space: 5Y/8/6) to yellow (Munsell Color Space 5Y/9/6) phenothiazine prills which met all standard product specifications, evidenced no precipitate, and generated efficacy similar to standard phenothiazine flake. The prills also offered improved handling and flowability.

The prills as formed were generally spherical in shape and had an average diameter of about 1 mm. The prills also had a low angle of repose and exhibited a very narrow particle size distribution. Low fines (powder) levels of less than 1% were achieved, and the product was not prone to caking. The product also evidenced flow, transfer and handling characteristics superior to the existing flake product. Additionally, due to the more uniform shape and smaller average particle size, the prills demonstrated an enhanced dissolution time in comparison to flake product. A comparison of the properties of standard flake and the prills formed in Example 2 are shown below in Table 1.

**Table 1**

| Properties | Flake | Prill (Ex 2) |
|---|---|---|
| Appearance | Yellow Flakes | Yellow Prills |
| Melting Point °C | 184 min. | 184.9 min. |
| Purity (%) | 99.6 | 99.9 |
| Angle of Repose | 36 | 25 |
| Bulk Density | 0.8 | 0.77 |
| Jar Shake Test for Powder | Slight to Heavy | Nil to Very Slight |
| Thickness (mm) | 1.40 | 1.65 |
| Average Diameter (mm) | N/A | 1 |
| Particle Size Distribution | | |
| % Particles ≥2360 microns | 75 | 0 |
| % Particles <2360 to 500 microns | 19 | 99 |
| % Particles <500 microns | 6 | 1 |
| Dissolution Time | | |
| Acetone (min.) | 5 | 3 |
| Methyl Methacrylate (min.) | 12.5 | 11 |
| Butyl Acrylate (min.) | 8 | 5.5 |
| Product Efficacy (hours to polymerize methyl methacrylate) | 13 | 14 |

With proper controls as herein described and an inert environment, preferably provided by liquid and gaseous nitrogen, it is possible to produce a prilled phenothiazine with the desired properties and color.

Based on the foregoing, prills of phenothiazine were formed which exhibited properties at least comparable, and in most cases superior to those of flaked and powdered phenothiazine. The prills also, advantageously, exhibited a substantially uniform average diameter and narrow particle size distribution as well as containing only a very low level of fines. Such characteristics provided reduced incidences of caking and/or clumping and demonstrated improved flow properties in transport and in use. Other significant advantages achievable by using these prills include improvements to environmental and workplace safety as well as a reduction in the cost of manufacturing resulting from the low level of fines.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

## Claims

1. A solid product comprising a phenothiazine material of formula (II) 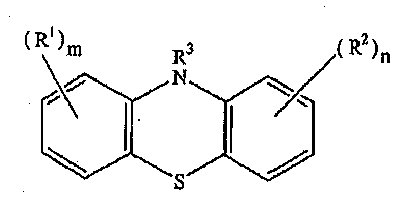 wherein R¹ R² and R³ are the same or different and are selected from the group consisting of hydrogen; halogen; branched and straight chain, and substituted and unsubstituted hydrocarbon groups selected from the group consisting of alkyl, alkenyl, and alkynyl groups of from 1 to 26 carbon atoms; substituted and unsubstituted aryl groups; substituted and unsubstituted aralkyl groups; sulfonyl; carboxy; amine; alkylamine; hydroxy; carboxy; silyl; siloxy; and derivatives and salts thereof; and m and n are independently from 1 to 4,
**characterised in that** the solid product is in the form of a plurality of generally spherical prills having an average diameter of from 0.5 mm to 2.3 mm, and that the solid product contains no greater than 6 % by weight of fines, i. e. particles with diameters of less than 500 µm.

2. A solid product according to claim 1, wherein the prills have a yellow colour within the region of the Munsell Colour Chart defined by Hue Symbol 5Y and Colour Spaces: 8/4 to 8/12, 8.5/4 to 8.5/12, 9/4 to 9/8, inclusive.

3. A solid product according to claim 1 or 2 wherein the yellow colour is within the region of the Munsell Colour Chart defined by Hue Symbol 5Y and Colour Spaces: 8.5/8 to 8.5/12 and 9/6 to 9/8, inclusive.

4. A solid product according to any one of claims 1 to 3 containing no more than 1 % by weight of fines.

5. A solid product according to any of the preceding claims wherein at least one of R¹ and R² is a moiety selected from the group consisting of a hydrocarbon group, an aryl group or an aralkyl group, and the moiety is substituted or interrupted by at least one member of the group consisting of oxygen, sulfur, silicon, nitrogen, sulfonyl, carboxy, amine, alkylamine, hydroxy, carboxy, silyl, and siloxy.

6. A solid product according to any one of claims 1 to 4 wherein each of R¹, R², and R³ is hydrogen.

7. A solid product according to claim 1 wherein the prills are formed by cooling molten droplets of phenothiazine in an inert atmosphere.

8. A solid product according to any one of claims 1 to 7 wherein the phenothiazine material is phenothiazine, and the prills are a greenish yellow to yellow colour.

9. A product according to claim 8 wherein the colour falls within the region of the Munsell Colour Chart defined by Hue Symbol 5Y and Colour Spaces: 8/4 to 8/12, 8.5/4 to 8.5/12 and 9/4 to 9/8, inclusive.

10. A product according to claim 8 wherein the colour falls within the region of the Munsell Colour Chart defined by Hue Symbol 5Y and Colour Spaces 8.5/8 to 8.5/12 and 9/6 to 9/8, inclusive.

11. A method for making a solid product as defined in claim 1 in prill form comprising breaking a stream of molten phenothiazine material into a plurality of evenly sized droplets and cooling the droplets to form solid prills of phenothiazine material, the phenothiazine material being maintained within an inert gas or liquid environment while in the molten state.

12. A method according to claim 11 wherein the inert gas or liquid environment is provided by nitrogen.

13. A method according to claim 11 or claim 12 wherein the molten phenothiazine is at a temperature not greater than 215 °C.

14. A method according to any one of claims 11 to 13 wherein the phenothiazine material is phenothiazine.

## Patentansprüche

1. Festes Produkt, bestehend aus einem Phenothiazin-Material der Formel (II), 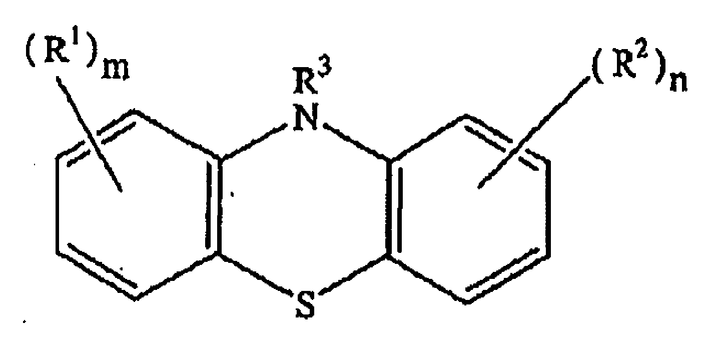 wobei R¹, R² und R³ gleich oder voneinander verschieden sind und ausgewählt sind aus der Gruppe bestehend aus Wasserstoff; Halogen; verzweigten und geradkettigen, und substituierten und unsubstituierten Kohlenwasserstoffgruppen, welche ausgewählt sind aus der Gruppe bestehend aus Alkyl-, Alkenyl- und Alkynylgruppen mit von 1 bis 26 Kohlenstoffatomen; substituierten und unsubstituierten Arylgruppen; substituierten und unsubstituierten Aralkylgruppen; Sulfonyl; Carboxy; Amin; Alkylamin; Hydroxy; Carboxy, Silyl; Siloxy und Derivaten und Salzen von diesen; und wobei m und n unabhängig voneinander Zahlen von 1 bis 4 sind,
**dadurch gekennzeichnet, dass** das feste Produkt in der Form einer Vielzahl von im Allgemeinen sphärischen Prills auftritt, die einen durchschnittlichen Durchmesser von 0,5 mm bis 2,3 mm aufweisen und dass das feste Produkt nicht mehr als 6 % des Gewichts an Feinstoffen enthält, d.h. Teilchen mit Durchmessern von weniger als 500 µm.

2. Festes Produkt nach Anspruch 1, wobei die Prills eine gelbe Farbe in dem Bereich der Munsell-Farbskala aufweisen, welcher durch Farbnuancensymbol 5Y und Farbfelder: 8/4 bis 8/12, 8,5/4 bis 8,5/12 und 9/4 bis einschließlich 9/8 definiert ist.

3. Festes Produkt nach Anspruch 1 oder 2, wobei die gelbe Farbe in den Bereich der Munsell-Farbskala fällt, welcher durch Farbnuancensymbol 5Y und Farbfelder: 8,5/8 bis 8,5/12 und 9/6 bis einschließlich 9/8 definiert ist.

4. Festes Produkt nach einem der Ansprüche 1 bis 3, das nicht mehr als 1 % des Gewichts an Feinstoffen enthält.

5. Festes Produkt nach einem der vorangehenden Ansprüche, wobei mindestens eines von R¹ und R² eine Baugruppe ist, welche ausgewählt ist aus der Gruppe bestehend aus einer Kohlenwasserstoffgruppe, einer Arylgruppe oder einer Aralkylgruppe, und wobei die Baugruppe substituiert oder unterbrochen ist durch mindestens ein Mitglied der Gruppe, welche aus Sauerstoff, Schwefel, Silicium, Stickstoff, Sulfonyl, Carboxy, Amin, Alkylamin, Hydroxy, Carboxy, Silyl und Siloxy besteht.

6. Festes Produkt nach einem der Ansprüche 1 bis 4, wobei R¹, R² und R³ ein Wasserstoff ist.

7. Festes Produkt nach Anspruch 1, wobei die Prills durch Kühlen geschmolzener Phenothiazin-Tröpfchen in einer inerten Atmosphäre gebildet werden.

8. Festes Produkt nach jedem der Ansprüche 1 bis 7, wobei das Phenothiazin-Material Phenothiazin ist und die Prills eine grünlich-gelbe bis gelbe Farbe aufweisen.

9. Produkt nach Anspruch 8, wobei die Farbe in dem Bereich der Munsell-Farbskala liegt, welcher durch Farbnuancensymbol 5Y und Farbfelder: 8/4 bis 8/12, 8,5/4 bis 8,5/12 und 9/4 bis einschließlich 9/8 definiert ist.

10. Produkt nach Anspruch 8, wobei die Farbe in dem Bereich der Munsell-Farbskala liegt, welcher durch Farbnuancensymbol 5Y und Farbfelder: 8,5/8 bis 8,5/12 und 9/6 bis einschließlich 9/8 definiert ist.

11. Verfahren zur Herstellung eines festen Produkts wie in Anspruch 1 definiert, bestehend aus Aufspalten eines Stroms geschmolzenen Phenothiazin-Materials in eine Vielzahl von Tröpfchen gleichmäßiger Größe und Kühlen der Tröpfchen zur Bildung von festen Prills aus Phenothiazin-Material, wobei das Phenothiazin-Material innerhalb eines Inertgases oder einer flüssigen Umgebung gehalten wird, während es sich im geschmolzenen Zustand befindet.

12. Verfahren nach Anspruch 11, wobei das Inertgas oder die flüssige Umgebung durch Stickstoff bereitgestellt wird.

13. Verfahren nach Anspruch 11 oder 12, wobei das geschmolzene Phenothiazin bei einer Temperatur von nicht höher als 215 °C vorliegt.

14. Verfahren nach einem der Ansprüche 11 bis 13, wobei das Phenothiazin-Material Phenothiazin ist.

## Revendications

1. Produit solide comprenant une substance de type phénothiazine de formule (II) 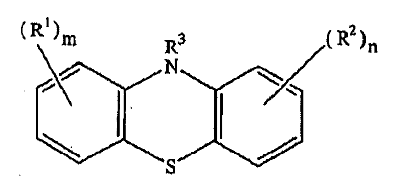 où R¹, R² et R³ sont identiques ou différents et sont choisis dans le groupe constitué par l'atome d'hydrogène ; les atomes d'halogène ; les groupements hydrocarbures linéaires ou ramifiés et éventuellement substitués, par exemple les groupements alkyle, alcényle ou alcynyle comportant entre 1 et 26 atomes de carbone ; les groupements aryle éventuellement substitués; les groupements arylalkyle éventuellement substitués ; les groupements sulfonyle, carboxy, amine, alkylamine, hydroxy, carboxy, silyle, siloxy; et leurs dérivés et sels ; et m et n sont indépendamment égaux à un nombre compris entre 1 et 4,
**caractérisé en ce que** le produit solide se présente sous forme d'une multitude de sphérules globalement sphériques de diamètre moyen compris entre 0,5 mm et 2,3 mm, et **en ce que** le produit solide ne contient pas plus de 6 % en masse de fines, c'est-à-dire de particules de diamètre inférieur à 500 µm.

2. Produit solide conforme à la revendication 1, où les sphérules présentent une couleur jaune située dans la région du nuancier de Munsell définie par la teinte 5Y et les espaces couleur : 8/4 à 8/12, 8.5/4 à 8.5/12, 9/4 à 9/8, inclus.

3. Produit solide conforme à la revendication 1 ou 2, où la couleur jaune est située dans la région du nuancier de Munsell définie par la teinte 5Y et les espaces couleur : 8.5/8 à 8.5/12 et 9/6 à 9/8, inclus.

4. Produit solide conforme à l'une quelconque des revendications 1 à 3 ne contenant pas plus de 1 % en masse de fines.

5. Produit solide conforme à l'une quelconque des revendications précédentes où au moins l'un des groupements R¹ et R² représente un groupement choisi dans le groupe constitué par un groupement hydrocarbure, un groupement aryle et un groupement arylalkyle, et le groupement est substitué ou interrompu par au moins un membre du groupe constitué par l'oxygène, le soufre, le silicium, l'azote, et les groupements sulfonyle, carboxy, amine, alkylamine, hydroxy, carboxy, silyle et siloxy.

6. Produit solide conforme à l'une quelconque des revendications 1 à 4 où chacun des groupements R¹, R² et R³ représente un atome d'hydrogène.

7. Produit solide conforme à la revendication 1 ou les sphérules sont formées par refroidissement de gouttelettes fondues de phénothiazine dans une atmosphère inerte.

8. Produit solide conforme à l'une quelconque des revendications 1 à 7 où la substance de type phénothiazine est la phénothiazine, et où les sphérules sont de couleur jaune verdâtre à jaune.

9. Produit conforme à la revendication 8, où la couleur jaune est incluse dans la région du nuancier de Munsell définie par la teinte 5Y et les espaces couleur : 8/4 à 8/12, 8.5/4 à 8.5/12 et 9/4 à 9/8, inclus.

10. Produit conforme à la revendication 8, où la couleur jaune est incluse dans la région du nuancier de Munsell définie par la teinte 5Y et les espaces couleur : 8.5/8 à 8.5/12 et 9/6 à 9/8, inclus.

11. Méthode de fabrication d'un produit solide conforme à la revendication 1 qui comprend la division d'un courant de substance de type phénothiazine fondue en une multitude de gouttelettes de même taille et le refroidissement de gouttelettes pour former des sphérules solides de substance de type phénothiazine, la substance de type phénothiazine étant conservée dans une atmosphère de gaz inerte ou un environnement liquide tant qu'elle est à l'état fondu.

12. Méthode conforme à la revendication 11, où l'atmosphère de gaz inerte ou l'environnement liquide sont formés d'azote.

13. Méthode conforme à la revendication 11 ou à la revendication 12, où la phénothiazine fondue est à une température ne dépassant pas 215 °C.

14. Méthode conforme à l'une quelconque des revendications 11 à 13, où la substance de type phénothiazine est la phénothiazine.
